Europäisches Patentamt

European Patent Office

Office européen des brevets

(11) Numéro de publication: **0 132 172**

**A1**

(12) # DEMANDE DE BREVET EUROPEEN

(21) Numéro de dépôt: **84401246.8**

(22) Date de dépôt: **18.06.84**

(51) Int. Cl.⁴: **C 10 G 29/00**
**C 07 C 11/02**

(30) Priorité: **22.06.83 FR 8310431**

(43) Date de publication de la demande:
**23.01.85 Bulletin 85/4**

(84) Etats contractants désignés:
**BE DE FR GB IT**

(71) Demandeur: **INSTITUT FRANCAIS DU PETROLE**
**4, Avenue de Bois-Préau**
**F-92502 Rueil-Malmaison(FR)**

(72) Inventeur: **Juguin, Bernard**
**46, Avenue de Stade**
**F-92500 Rueil-Malmaison(FR)**

(72) Inventeur: **Miquel, Jean**
**5, rue Fragonard**
**F-75017 Paris(FR)**

(54) **Procédé de production de supercarburant par polymérisation des coupes C4.**

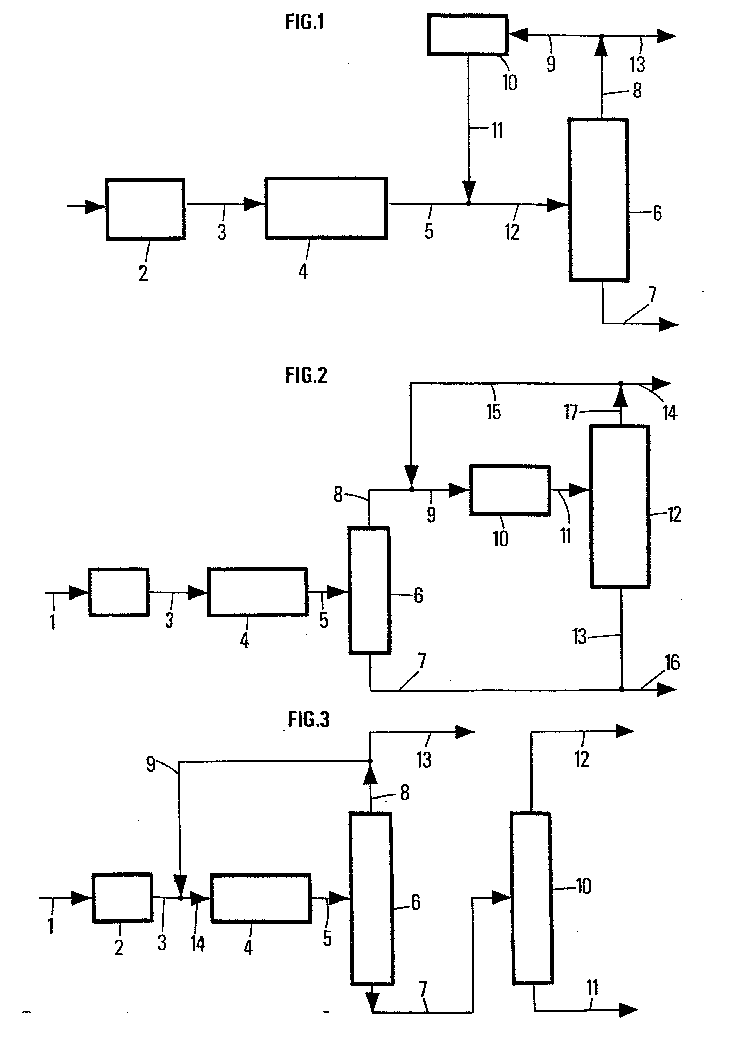

(57) L'invention concerne un procédé de production de dimères et trimères de l'isobutène à partir d'un mélange d'isobutène et de butènes. Elle est caractérisée en ce que (Figure 1) la charge est introduite dans une premiere zone catalytique (4) de polymérisation, en ce que l'effluent de polymérisation est fractionné de façon à éliminer les dimères et trimères de l'isobutène formés et en ce que l'isobutène et les butènes non transformés sont traités dans une seconde zone catalytique (10) de polymérisation fonctionnant dans des conditions opératoires différentes de la première zone de polymérisation.

FIG.1

EP 0 132 172 A1

1

# PROCEDE DE PRODUCTION DE SUPERCARBURANT PAR POLYMERISATION DES COUPES $C_4$.

La présente invention concerne un procédé de valorisation des coupes $C_4$ oléfiniques mises à la disposition du marché par le développement des divers procédés de craquage à la vapeur et le craquage catalytique des naphtas et des gazoles.

Les oléfines, butènes et isobutènes, peuvent être utilisés pour les synthèses pétrochimiques de produits plus élaborés (alcools, aldéhydes, acides, nitriles, etc...), mais les tonnages de ces coupes $C_4$ sont, en général, trop importants ou du moins peuvent devenir trop importants pour que cette première voie de valorisation de coupes $C_4$ puisse être seule envisagée.

Une deuxième voie de valorisation consiste à recycler cette coupe $C_4$ vers l'unité de craquage (craquage à la vapeur) ou de craquage catalytique, après hydrogénation des oléfines ; mais, ici, l'isobutane contenu dans la coupe hydrogénée, et qui représente plus de 50 % en poids de la coupe considérée, ne donne pas des rendements en éthylène suffisamment intéressants et conduit aussi à une production importante de méthane qui n'est guère utilisable que comme combustible.

Une troisième voie de valorisation de ladite coupe $C_4$ consiste, après hydrogénation de cette coupe, à séparer le n-butane de l'isobutane par distillation et à ne recycler vers l'unité de vapocraquage que le n-butane, réservant l'isobutane pour d'autres utilisations plus avantageuses, telles que par exemple une alkylation pour la production d'essences iso-paraffiniques. Néanmoins, les rendements en éthylène et propylène, dans un vapocraquage ainsi réalisé, n'excèdent pas 38 et 20 % respectivement, en poids, et la production de méthane avoisine 25 % en poids.

Une quatrième voie de valorisation de la coupe $C_4$ consiste à alkyler les oléfines de cette coupe avec l'isobutane qu'elle contient, de façon à maximiser les rendements en essences. Cependant, dans l'étape d'alkylation, le comportement de l'isobutène est moins favorable que celui des butènes pour l'obtention d'une essence à nombre d'octane élevé; en effet, les nombres d'octane Research (ou Clair) des alkylats de butènes normaux sont plus élevés que ceux des alkylats d'isobutène.

Il existe encore d'autres voies, par exemple celle consistant à transformer en di et trimères de l'isobutène, l'isobutène de la charge sans toucher sensiblement aux butènes de la charge, à isomériser ensuite le butène-1 de la charge en butène-2 en vue de faire subir ensuite aux butènes-2 une réaction d'alkylation par de l'isobutane ( brevet français N° 2 492 635).

Une autre méthode consisterait à modifier cette dernière technique : la méthode consiste à transformer la totalité des butènes présents dans la charge initiale en dimères et trimères de l'isobutène, lesquels produits constituent un excellent carburant automobile.

Pour effectuer cette méthode (voir par exemple la demande de brevet français N° 81/20373 du 28 octobre 1981), on soumet la charge (coupe $C_4$ oléfinique) à une polymérisation au cours de laquelle l'isobutène est converti, à 90 % en poids ou plus, en dimères et trimères, que l'on sépare des butènes non transformés par un simple fractionnement et que l'on envoie au pool essence. On sépare alors les butènes normaux, (ceux-ci n'ayant pas ou peu réagi au cours de la polymérisation)et on en extrait les hydrocarbures paraffiniques en $C_4$ que l'on peut envoyer vers le pool essence. La fraction ayant subi l'extraction est très riche en n-butènes (au moins 99 % en poids) et , par isomérisation, on transforme ces n-butènes en isobutène lequel, à son tour, sera transformé en dimères et trimères de l'isobutène.

De cette manière, on peut donc convertir en essence la totalité de la coupe $C_4$ oléfinique initiale.

Or on a maintenant découvert qu'il pouvait être judicieux de modifier cette dernière technique de la façon suivante qui consiste aussi à transformer la majeure partie des butènes contenus dans la charge initiale en dimères et trimères des butènes de départ, lesquels sont connus comme étant des produits qui constituent un excellent carburant automobile . (On transforme ainsi,à l'issue du procédé,au moins 90 % du butène-1, au moins 80 % du butène-2 et au moins 97 % de l'isobutène de la coupe $C_4$ oléfinique initiale sans obtenir sensiblement de kérosène).

Le procédé faisant l'objet de la présente invention consiste ainsi à soumettre d'abord la charge (coupe $C_4$ oléfinique), charge généra-lement débarrassée d'au moins la majeure partie du butadiène qu'elle peut contenir, à une première réaction de polymérisation au cours de laquelle les conversions globales des butènes normaux contenus dans la charge restent inférieures à 45 % en poids et au cours de laquelle on convertit au moins 80 % en poids de l'isobutène présent (de préférence, on effectue au moins 85 % de conversion de l'isobutène), ces hydrocarbures se transformant essentiellement en dimères et trimères ; ensuite, le produit sortant du réacteur est soumis à un fractionnement en vue d'obtenir, d'une part, une première fraction, contenant de l'isobutène et des butènes n'ayant pas réagi (que l'on envoie au moins en partie vers une deuxième (différente de la première zone de polymérisation) zone de polymérisation /,dans laquelle les conditions opératoires sont optimisées pour transformer les butènes normaux en dimères et trimères, opération que l'on décrira plus loin) et d'autre part, une deuxième fraction constituée essentiellement par les dimères et trimères (de l'isobutylène) que l'on envoie au pool essence.

Ainsi,ladite première fraction très riche en butènes normaux, n'ayant pas réagi, ainsi que le reste de l'isobutène non transformé, sont envoyés vers un deuxième réacteur d'isomérisation où les oléfines présentes sont transformées en majeure partie en dimères et trimères des oléfines présentes.On convertit ici pratiquement à 100% l'isobutène présent et au moins 50 % en poids des butènes-1 et 2 et de préférence on effectue, dans ce réacteur au moins 75 % en poids de conversion du butène-1 et plus de 55 % en poids de conversion des butènes-2.

On peut opérer selon deux variantes :dans une première de procéder, le produit sortant de ce deuxième réacteur de polymérisation, contenant encore conjointement avec les dimères et trimères produits, une certaine quantité d'oléfines en $C_4$ qui n'ont pas réagi, est envoyé conjointement avec le produit sortant du premier réacteur de polymérisation vers la colonne de fractionnement afin de séparer les dimères et trimères obtenus des oléfines en $C_4$ qui n'ont pas réagi.

La deuxième manière de procéder, également conforme à la présente invention, consiste à envoyer le produit sortant du deuxième réacteur de polymérisation fonctionnant comme précédemment vers une deuxième colonne de fractionnement/,où l'on obtient, d'une part, une
distincte de la première colonne/
première fraction, contenant les butènes normaux n'ayant pas réagi (dont la majeure partie est recyclée vers le deuxième réacteur de polymérisation, après en avoir prélevé une partie aliquote, inférieure à environ 20 % en poids de la dite fraction, que l'on envoie vers le pool essence, le but de cette opération, étant celui de faciliter l'élimination des hydrocarbures paraffiniques apportés par la charge) et d'autre part, une deuxième fraction constituée par les dimères et trimères des butènes (butènes normaux et isobutène n'ayant pas réagi dans le premier réacteur de polymérisation) que l'on envoie au pool essence.

Le procédé correspondant à l'invention, avec ses deux variantes, est schématisé dans les deux figures 1 et 2 qui suivent

Première variante (figure 1) : Une coupe $C_4$ oléfinique, qui généralement à ce stade renferme de l'isobutane, du n-butane, du butène-1, des butènes-2, de l'isobutène, et un peu ou pas de propène et ou du butadiène (en général moins de 2 % et de préférence moins de 0,7 % en poids de propène et de butadiène) est introduite par la conduite 1 dans une zone éventuelle de séchage 2. Ce séchage peut être effectué de façon conventionnelle, par exemple par passage de la coupe, sur une alumine, un tamis moléculaire, de préférence un tamis de type 3 A. La coupe ainsi séchée est envoyée par la conduite 3 dans une zone de polymérisation 4 au cours de laquelle la majeure partie de l'isobutène contenu dans la charge est essentiellement transformée en dimères et trimères de l'isobutène.

Dans cette zone de polymérisation, les conditions opératoires sont telles que l'isobutène réagit jusqu'à des taux de conversion supérieurs à 80 % voire à 82 % en poids, de préférence 85 % et même 95 %, tandis que les conversions globales des butènes normaux restent inférieures à 45 % en poids.

Les réactions de polymérisation sont en général effectuées en présence d'un catalyseur disposé par exemple sous forme d'un lit fixe à une température d'environ 40 à 250°C (environ), sous une pression d'environ 0,1 à 10 MPa (1 à 100 bars) / de préférence la température est d'environ 45 à 200°C et la pression est de 0,1 à 5 MPa ; le débit d'hydrocarbures liquides (vitesse spatiale) est d'environ 0,05 à 5 volumes par volume de catalyseur et par heure, de préférence entre 0,8 et 2,5.

Pour cette première zone de polymérisation 4, les conditions opératoires seront plus douces, que dans la deuxième zone 10, afin d'empêcher une polymérisation plus profonde de l'isobutène, c'est-à-dire afin d'éviter la production d'hydrocarbures ayant plus de 12 atomes de carbone par molécule.

Le catalyseur de nature acide peut être une silice alumine ou une bore alumine ou alumine borée. On peut encore choisir un catalyseur obtenu par traitement d'alumine de transition au moyen d'au moins/ un dérivé acide du fluor, avec éventuellement addition d'un ester silicique. Les catalyseurs utilisés selon la présente invention, pour la réaction de polymérisation, se révèlent avoir des qualités supérieures à celles d'autres catalyseurs de polymérisation tels que l'acide sulfurique ou encore l'acide phosphorique. Ces types de catalyseur sont utilisés dans USP 2,298,330 mais conduisent au cours de la polymérisation de l'isobutène à la formation parasite d'essence lourde. Lorsqu'on utilise de l'acide phosphorique, cet acide peut déposé sur Kieselguhr ou sur silice, ou sur quartz , ou peut être du type "acide phosphorique solide", (catalyseur constitué d'une matière silicieuse à grand pouvoir absorbant imprégnée d'une proportion élevée d'acide phosphorique).

De préférence, on utilise dans la présente invention, une silice alumine dont la teneur en silice est comprise entre 60 et 95 % en poids et de préférence entre 70 et 90 %, pouvant avoir comme additif entre 0,1 et 0,5 % d'oxyde de chrome ou/et oxyde de zinc. La surface spécifique de la silice alumine utilisée peut avantageusement être comprise entre 50 et 500 $m^2$ par gramme, de préférence entre 150 et 400$m^2$/g, son volume poreux étant compris, par exemple, entre 0,40 et 0,90 $cm^3$ par gramme.

L'effluent de polymérisation est soutiré par les conduites 5 et 12.

Après séparation, par distillation, dans la colonne 6, des dimères et trimères (de l'isobutène et des butènes) obtenus que l'on envoie par la conduite 7 vers le pool essence, on recueille par la conduite 8 l'isobutène et les butènes n'ayant pas réagi, ainsi que les hydrocarbures paraffiniques contenus dans la charge brute, une première partie (qui ne sera généralement pas supérieure à 30 % en poids des hydrocarbures en $C_4$ sortant en tête de colonne) est envoyée par la conduite 13 vers le pool essence, le but de cette séparation étant d'éliminer l'excédent des hydrocarbures paraffiniques, qui autrement s'accumuleraient dans les gaz de recyclage et une deuxième partie, la plus importante est envoyée, par la conduite 9, dans une deuxième zone de polymérisation 10, dans laquelle les conditions de pression et température sont plus sévères que celles de la première zone (4) de polymérisation, (c'est-à-dire que, la température d'entrée et la température de sortie pour la deuxième zone de polymérisation sont chacune supérieure de 20 à 60°C, et de préférence 30 à 50°C, aux température d'entrée et de sortie de la première zone de polymérisation et la pression dans la deuxième zone de polymérisation est supérieure de 0,4 à 3 MPa et de préférence 0,8 à 2 MPa à la pression dans la première zone de polymérisation) et où les /de l'isobutène et des butènes/

oléfines en $C_4$ présentes sont transformées en dimères et trimères/: les taux de conversion de l'isobutène résiduel est de 100 % ; la conversion du butène-1 peut atteindre 80 %, tandis que les conversions globales des butènes-2 ne dépassent pas 60 % en poids.

Les produits obtenus ainsi que les gaz n'ayant pas réagi sortant de la zone de polymérisation 10 sont envoyés, par la conduite 11, conjointement avec les produits sortant, par la conduite 5, de la première zone de polymérisation 4, vers la zone de distillation 6.

Deuxième variante (figure 2) : Les éléments 1 à 7 de cette figure 2 sont définis comme les éléments 1 à 7 de la figure 1. Dans cette variante, les produits gazeux sortant de la zone de fractionnement 6, par les conduites 8 et 9, sont envoyés en totalité dans la deuxième zone de polymérisation 10 où les conversions sont du même ordre de grandeur que dans le cas de la variante 1. Les produits après réaction, sortant de cette zone par la conduite 11, au lieu d'être recyclés, comme dans la première variante, vers la zone de distillation 6, sont introduits dans une deuxième zone de distillation 12, dans laquelle les dimères et trimères des oléfines en $C_4$ obtenus sont séparés des hydrocarbures parafiniques et des oléfines n'ayant pas réagi, et envoyés par les conduites 13 et 16 vers le pool essence, conjointement avec les dimères et trimères provenant de la colonne de distillation 6 par la conduite 7.

Les gaz sortant en tête de la colonne 12 et constitués exclusivement par les butènes résiduaires ainsi que les hydrocarbures paraffiniques introduits par la charge initiale sont divisés en deux parties. Une première partie (qui ne sera pas supérieure à 30 % en poids des hydrocarbures en $C_4$ sortant en tête de colonne) est envoyée, par les conduites 17 et 14 vers le pool essence. Comme dans la première variante le but de cette séparation étant d'éliminer l'excédent des hydrocarbures paraffiniques. La deuxième partie, la plus importante, est recyclée par la conduite 15 vers la deuxième zone de polymérisation 10.

Compte-tenu de la forte exothermicité à laquelle donne lieu la transformation de polymérisation dans la zone 4, il sera préférable que la teneur en isobutène de la charge ne soit pas supérieure à environ 45 % en poids, sinon il conviendra de la diluer, par exemple avec du butane ou de l'isobutane et/ou par exemple à l'aide d'une partie, ou de la totalité des hydrocarbures en $C_4$ riches en butanes, en provenance par la conduite 13, dans le cas de la première variante, ou par la conduite 14 pour la deuxième variante, de l'effluent séparé en tête des colonnes de distillation 6 ou 12 respectivement.

Cette fraction recyclée riche en butanes sera envoyée dans la première zone de polymérisation 4 dans les deux cas (figures 1 et 2).

, utiliser/

On peut aussi pour effectuer la dilution/de l'isobutane (et/ou du butane) frais.

Exemple 1 : (illustrant la figure 1)

A titre d'exemple, on se propose de traiter une coupe $C_4$ oléfinique de vapocraquage après extraction du butadiène.

La composition de cette charge est donnée, en pourcent en poids, dans le tableau I.

TABLEAU I

Composition de la charge (% en poids)

| | |
|---|---|
| Propyne | 0,05 |
| Isobutane | 1,47 |
| n-butane | 7,89 |
| isobutène | 39,42 |
| butène-1 | 28,76 |
| ≤ butènes-2 | 22,15 |
| butadiène 1-3 | 0,26 |

La charge est d'abord soumise à un séchage sur un tamis moléculaire 3 A, (zone 2 de la figure), puis elle est envoyée par la conduite 3 dans une zone 4 de polymérisation des butènes.

La réaction de polymérisation a lieu en présence d'un catalyseur qui est une silice-alumine à 90 % en poids de silice, ayant une surface spécifique de $420 m^2/g$ et un volume poreux de 0,40 $cm^3/g$.

Les conditions opératoires sont les suivantes :

Pression                              : 1,2 MPa
Température à l'entrée du réacteur  : 55°C
Température à la sortie du réacteur : 75°C
Vitesse spatiale (VVH)              : 2 $h^{-1}$

La réaction se déroule en phase liquide.

L'effluent obtenu est soumis à un fractionnement (zone 6 de la figure) conjointement avec les produits recyclés arrivant par la conduite 11 en provenance du deuxième réacteur de polymérisation 10.

En fond de colonne, par la conduite 7, on recueille un polymérisat, qui représente 84,28% en poids par rapport à la charge initiale, constituée exclusivement par des dimères et trimères des hydrocarbures /en C4/ dont 45,03 % ont été produits dans la zone de polymérisation 4, le complément à 84,28 % c'est-à-dire 39,25 % provenant des produits obtenus dans la deuxième zone de polymérisation 10. On notera l'absence de polymères à plus de 12 atomes de carbone par molécule.

En tête de colonne, par la conduite 8, on obtient une fraction ayant la composition suivante en pourcent en poids par rapport à la charge initiale :

| | |
|---|---|
| Butane + isobutane | 93,60 |
| Isobutène | 5,12 |
| Butène-1 | 22,45 |
| Σ butènes-2 | 35,88 |
| Butadiène 1-3 | 0,08 |

De cet effluent gazeux, on en prélève 10 % en poids de la quantité totale sortant en tête de colonne afin d'éviter une accumulation excessive d'hydrocarbures en $C_4$ paraffiniques dans la boucle de recyclage. Le dit prélèvement est envoyé, par la conduite 13, vers le pool essence, mais il peut aussi être recyclé vers la zone de polymérisation 4 dans le cas où il serait nécessaire de modérer l'exothermicité de la réaction.

Le 90 % en poids de l'effluent qui reste et contenant encore des quantités non négligeables de butènes, lesquels dans le présent exemple s'élèvent à 40,38 % en poids de l'effluent, est envoyé, par la conduite 9, dans une deuxième zone de polymérisation 10, contenant le même catalyseur que la première, 4, mais dans laquelle les conditions opératoires sont plus sévères pour favoriser la transformation des butènes à chaîne droite (butènes-1 et -2).

Dans ce deuxième réacteur 10, les conditions opératoires sont les suivantes :

| | |
|---|---|
| Pression | : 2,5 MPa |
| Température à l'entrée du réacteur | : 95°C |
| Température à la sortie du réacteur | : 115°C |
| Vitesse spatiale (VVH) | : 1 h$^{-1}$ |

La réaction ici a lieu aussi en phase liquide.

Le produit de la réaction sortant du réacteur 10, par la conduite 11 ne contient plus d'isobutène ni de butadiène, lesquels ont été complètement transformés en polymères et a la composition suivante en pourcent en poids par rapport à la charge de départ.

| | |
|---|---|
| Butane + isobutane | : 84,24 |
| Butène-1 | : 4,04 |
| ≤ butènes-2 | : 13,88 |
| Dimères et trimères | : 39,25 |
| de l'isobutène et des n-butènes | |

0132172

11

Cet effluent est envoyé conjointement avec celui provenant du premier réacteur de polymérisation 4 vers la zone de fractionnement 6. Au cours de cette deuxième étape pratiquement 100 % de l'isobutène, environ 78 % du butène-1 et 58 % du butène-2 de la charge initiale ont été convertis en dimères et trimères de l'isobutène et des butènes normaux.

Les principales caractéristiques de la coupe essence obtenue en fond de la colonne de fractionnement 6, qui est envoyée, par la conduite 7, vers le pool essence, et qui représente 84,28 % en poids de la charge de départ.

| | |
|---|---|
| Densité à 20°C | : 0,739 |
| Indice de brome | : 111 |
| Indice d'octane NOR clair | : 99,5 |
| - Distillation ASTM | |
| PI | : 60°C |
| 5 | : 104°C |
| 10 | : 108°C |
| 20 | : 109°C |
| 30 | : 111°C |
| 40 | : 113°C |
| 50 | : 118°C |
| 60 | : 125°C |
| 70 | : 137°C |
| 80 | : 156°C |
| 90 | : 189°C |
| 95 | : 193°C |
| PF | : 213°C |
| Distillat | : 100 % |
| Résidu | : 0 |
| Pertes | : 0 |

On rappelle ici les spécifications concernant les points finals des essences.

| | |
|---|---|
| JAPON | : 225°C |
| U.S.A. | : 220°C |
| FRANCE | : 215°C |

12

Exemple 2 : (illustrant la figure 2)

Ici la charge à traiter est la même que celle utilisée dans l'exemple précédent.

Le catalyseur utilisé est le même. Les conditions opératoires dans les deux réacteurs de polymérisation 4 et 10 sont aussi les mêmes que celles de l'exemple précédent.

La seule différence réside dans la disposition de l'appareillage utilisé. En effet, dans cet exemple, on utilise une colonne de distillation supplémentaire 12 permettant la distillation indépendante des effluents des réacteurs 4 et 10. Le volume de produit recyclé se trouvant ainsi diminué.

Cette disposition demande l'emploi de deux colonnes de distillation, elles seront en conséquence généralement de taille nettement réduites par rapport à la colonne de la figure 1.

L'effluent sortant de la zone de polymérisation 4, est envoyé, par la conduite 5, dans la colonne de fractionnement 6. En fond de colonne, par la conduite 7, on recueille un polymérisat, qui représente 45,03 % en poids de la charge initiale /et/ qui est une essence de qualité supercarburant.

En tête de colonne, par la conduite 8, on obtient une fraction ayant la composition suivante en pourcent en poids par rapport à la charge initiale.

| | | |
|---|---|---|
| Butane + isobutane | : | 9,36 % |
| Isobutène | : | 5,12 % |
| Butène-1 | ; | 18,41 % |
| ∠Butènes-2 | : | 22,15 % |
| Butadiène 1-3 | : | 0,08 % |

Cet effluent constitué exclusivement par des hydrocarbures en $C_4$ est envoyé, par la conduite 8, dans une deuxième zone de polymérisation 10 conjointement avec un deuxième effluent constitué par les hydrocarbures en $C_4$ séparés en tête de la deuxième colonne de distillation 12 et arrivant par la conduite 15.

deuxième/

Ce effluent,ne contenant que des butènes normaux et des butanes,a la composition suivante, en pourcent en poids par rapport à la charge initiale :

| | | |
|---|---|---|
| Butane + isobutane | : | 37,20 % |
| Butène-1 | : | 3,45 % |
| ∠Butènes-2 | : | 11,52 % |

Le produit sortant de la deuxième zone de polymérisation 10, est envoyé, par la conduite 11, vers une deuxième colonne de fractionnement 12.

En fond de colonne, par la conduite 13, on recueille un polymérisat, qui représente 41,83% en poids de la charge initiale, qui est cons-/en C4 et est envoyé,/ titué exclusivement par des dimères et trimères des hydrocarbures oléfiniques/ conjointement avec le polymérisat provenant du fond de la colonne 6 (constituant 45,03 % du poids de la charge initiale),vers le pool essence.

Ainsi on obtient en tout 86,86 % en poids d'essence qualité supercarburant.

En tête de colonne, on prélève un effluent ayant la composition suivante, en pourcent en poids par rapport à la charge initiale :

| Butane + isobutane | : | 41,77% |
|---|---|---|
| Butène-1 | : | 4,37% |
| ≤ Butènes-2 | : | 14,42% |

De cet effluent, on prélève avant recyclage, par la conduite 15, vers le deuxième réacteur de polymérisation 10, 20 % en poids du produit total sortant en tête de colonne, ce qui représente 13,14 % en poids de la charge initiale. La composition de cet effluent que l'on envoie vers le pool essence, a la composition suivante en pourcent en poids :

| Butane + isobutane | : | 71,23% |
|---|---|---|
| Butène-1 | : | 6,85% |
| ≤ Butènes-2 | : | 21,92% |

100 %

Les 80 % en poids restant de cet effluent qui représentent 52,17 % en poids de la charge initiale et qui contiennent 28,77 % en poids de butènes polymérisables sont recyclés, par la conduite 15, vers la deuxième zone de polymérisation 10, où ils s'ajoutent à l'effluent arrivant par la conduite 8 provenant de la première colonne de distillation 6.

Au cours de cette deuxième étape, pratiquement 100 % de l'isobutène, environ 80 % du butène-1 et 60 % du butène-2 de la charge initiale ont été convertis en dimères et trimères de l'isobutène.

Exemple 3 comparatif : (illustré par la figure 3)

Comme on peut voir sur la figure 3 illustrant la manière usuelle utilisée pour polymériser les butènes, on utilise ici un réacteur unique.

La charge et le catalyseur utilisés sont les mêmes que ceux employés dans les deux exemples précédents.

La charge (conduite 1) est d'abord séchée par passage à travers un tamis moléculaire 3 A (zone 2 de la figure) puis elle est envoyée par les conduites 3 et 14, conjointement avec les hydrocarbures recyclés arrivant par la conduite 9, dans la zone de polymérisation 4.

Dans ce réacteur, les conditions opératoires sont, dans le cas présent, les suivantes :

| | |
|---|---|
| Pression | : 2,5 MPa |
| Température à l'entrée du réacteur | : 95°C |
| Température à la sortie du réacteur | : 115°C |
| Vitesse spatiale (VVH) | : $1\ h^{-1}$ |

La réaction a lieu ici aussi en phase liquide.

Le produit sortant de la zone de réaction 4, est envoyé par la conduite 5, dans une première colonne de distillation 6, où il est soumis à un fractionnement.

En fond de colonne, par la conduite 7, on recueille un polymérisat, qui représente 87,67 % en poids de la charge initiale, contenant en plus des dimères et trimères de constituants plus lourds.

16

Les caractéristiques de ce produit sont les suivantes :

| | | |
|---|---|---|
| Densité (20°C) | : | 0,763 |
| Indice de brome | : | 82 |
| Indice d'octane NOR Clair | : | 97,5 |
| -Distillation ASTM | | |
| PI | : | 78°C |
| 5 | : | 112°C |
| 10 | : | 116°C |
| 20 | : | 121°C |
| 30 | : | 128°C |
| 40 | : | 138°C |
| 50 | : | 151°C |
| 60 | : | 169°C |
| 70 | : | 185°C |
| 80 | : | 203°C |
| 90 | : | 238°C |
| 95 | : | 249°C |
| PF | : | 270 |
| Distillat | : | 98 % |
| Résidu | : | 2 % |
| Pertes | : | 0 |

17

Les Normes Françaises limitent le point final des essences pour automobile à 215°C, il sera donc nécessaire de soumettre ce produit à un fractionnement décrit plus loin.

En tête de colonne, par la conduite 8, on obtient une fraction, constitué par les paraffines et oléfines en $C_4$ n'ayant pas réagi, dont la composition est la suivante en pourcent par rapport à la charge initiale :

|  |  |  |
|---|---|---|
| Butane + isobutane | : | 93,36 |
| Butène-1 | : | 1,16 |
| $\leq$ Butènes-2 | : | 28,46 |

De cet effluent, on en prélève 10 % en poids, ce qui permet de purger les paraffines en $C_4$ qui autrement, ne réagissant pas, s'accumuleraient sans discontinuer.

Cette purge, surtout par la conduite 13, très riche en butanes, (elle en contient 75,91 % en poids), est envoyée vers le pool essence.

Les 90 % qui restent sont recyclés, par la conduite 9, vers le réacteur de polymérisation 4 où ils rejoignent la charge fraîche.

Le produit récupéré en fond de colonne (zone de distillation 6), qui est non conforme aux spécifications françaises pour le point final de distillation ASTM, est envoyé par la conduite 7 dans une deuxième zone de distillation 10.

En tête de cette colonne, on obtient une coupe supercarburant, représentant 72,76 % en poids de la charge fraîche, que l'on envoie, par la conduite 12, au pool essence.

En fond de colonne, par la conduite 11, on soutire une coupe kérosène, représentant 14,91 % en poids de la charge de départ, laquelle après hydrogénation pourra être utilisée comme combustible pour carburéacteur.

18

Tableau comparatif : Rendements exprimés en pourcent en poids par rapport à la charge.

TABLEAU I

| EXEMPLE | 1 | 2 | 3 (comparatif) |
|---|---|---|---|
| Hydrocarbures en $C_4$ envoyés vers le pool essence | 15,72 | 13,14 | 12,33 |
| Supercarburant (dimères et trimères de l'isobutène) | 84,28 | 86,86 | 72,76 |
| Kérosène (à hydrogéner) | - | - | 14,91 |
| TOTAL | 100 | 100 | 100 |

On constate que les procédés selon les figures 1 et 2 donnent des rendements nettement plus importants en supercarburant directement utilisable, qu'en opérant selon le procédé de la figure 3 (comparatif) classique.

L'utilisation de deux réacteurs successifs permet d'optimiser les conditions opératoires dans chacun d'eux, ce qui permet d'éviter, comme c'est le cas en utilisant un seul réacteur, une polymérisation trop poussée de l'isobutène conduisant à la production d'hydrocarbures trop lourds inutilisables comme carburant automobile.

Dans le tableau II, on présente les taux totaux de conversion des oléfines pour chacun des trois exemples.

.TABLEAU II

| EXEMPLE | 1 | 2 | 3 |
|---|---|---|---|
| Propyne | 100 % | 100 % | 100 % |
| Isobutène | 98,71 | 100 % | 100 % |
| Butène-1 | 92,18 | 96,87 | 96,1 |
| Butènes-2 | 83,79 | 87 | 40,4 |
| Butadiène 1-3 | 96,15 | 100 | 100 |

Exemple 4 (comparatif)

On répète l'exemple 2 dans les mêmes conditions opératoires. Toutefois, on utilise comme catalyseur dans chacune des 2 zones de polymérisation (4) et (10), non plus de la silice alumine, mais un mélange solide d'acide phosphorique et de silice (renfermant en poids 68% de $P_2O_5$ et 32% de $SiO_2$), ce catalyseur se présentant sous forme d'extrudés de 5 à 6mm de diamètre, avec les caractéristiques suivantes :

- densité de grain              :    1,99
- densité structurale           :    2,99
- volume poreux total           :    0,11 $cm^3/g$
- surface spécifique            :    6,5  $m^2/g$

Au démarrage, les conversions de l'isobutène en dimères et trimères dans la première zone (4) de polymérisation sont correctes et du même ordre de grandeur que dans l'exemple 2 mais dans la deuxième zone de polymérisation (10), le taux de conversion des n-butènes est faible de sorte que le polymérisat, soutiré par la conduite (13) de la zone (12) de fractionnement, ne constitue que 23,50% du poids total de la charge au lieu de 45,03% dans l'exemple 2.

Puis, rapidement, le catalyseur des deux zones de polymérisation (4) et (10), perd son activité en raison de la formation de phosphate d'alkyle qui entraîne une démolition rapide du catalyseur par entraînement de l'acide phosphorique.

Pour remédier à cet inconvénient, il conviendrait alors d'opérer à 200°C dans chacune des deux zones de polymérisation et sous une pression de 4 MPa (c'est-à-dire sous des conditions peu souhaitables), ces conditions opératoires permettent d'obtenir un polymérisat représentant 77,15% par rapport au poids de la charge initiale (86,86% dans l'exemple 2). Mais on observe alors la formation de 9,71% (pour l'ensemble des deux zones (4) et (10) de polymérisation) en poids par rapport à la charge initiale, d'un produit lourd de point d'ébullition supérieur à 235°C constitué de polymères en $C_{12}^+$ de l'isobutène et des n-butènes.

1

## REVENDICATIONS

1.- Procédé de valorisation d'une coupe $C_4$ oléfinique renfermant des butènes-1 et -2 et de l'isobutène et débarrassée d'au moins la majeure partie du butadiène qu'elle pourrait contenir, le procédé consistant à convertir en dimères et trimères des hydrocarbures en C4 de la charge au moins 97% de la totalité de l'isobutène de ladite charge et au moins la majeure partie des butènes-1 et -2, (et sans obtenir des quantités sensibles de kérosène) ledit procédé étant caractérisé en ce que dans une première étape, on traite la coupe $C_4$ oléfinique dans une première zone catalytique dite de polymérisation à une température d'entrée et de sortie comprise entre 40 et 250°C, sous une pression comprise entre 0,1 et 10 MPa avec un débit d'hydrocarbures liquides (vitesse spatiale) d'environ 0,05 à 5 volumes par volume de catalyseur et par heure, en présence d'un catalyseur choisi dans le groupe constitué par les silice-alumines, les bore-alumines, les alumines borées et les alumines de transition traitées par au moins un dérivé acide du fluor, de façon à convertir au moins 80% de l'isobutène en dimères et trimères de l'isobutène et à ne pas convertir plus de 45% du mélange des butènes-1 et 2 en dimères et trimères de ces hydrocarbures, en ce que ensuite on fractionne l'effluent de ladite première zone catalytique de polymérisation de façon à recueillir d'une part en fond de colonne essentiellement les dimères et trimères des hydrocarbures oléfiniques en C4 et d'autre part en tête de colonne un mélange renfermant la majeure partie de l'isobutène et des butènes-1 et -2 qui n'ont pas été convertis dans ladite première zone catalytique de polymérisation, ledit mélange étant au cours d'une deuxième étape au moins en partie envoyé dans une deuxième zone catalytique de polymérisation fonctionnant à une température d'entrée et de sortie comprise entre 40 et 250°C, sous une pression comprise entre 0,1 et 10 MPa et avec un débit d'hydrocarbures liquide (vitesse spatiale) d'environ 0,05 à 5 volumes par volume de catalyseur et par heure, les températures d'entrée et de sortie pour la deuxième zone de polymérisation étant chacune supérieure de 20 à 60°C aux températures d'entrée et de sortie de la première zone de polymérisation, la pression dans la deuxième zone de polymérisation étant supérieure de 0,4 à 3 MPa à la pression dans la première zone de polymérisation, en présence d'un catalyseur choisi dans le groupe constitué par les silice-alumines, les bore-alumines, les alumines borées et les alumines de transition traitées par au moins un dérivé acide du fluor, de façon à convertir a) en dimères et trimères de l'isobutène, sensiblement, dans cette dite deuxième zone, la totalité de l'isobutène et b) en dimères et trimères des n-butènes, au moins 50% des butènes-1 et-2 qui sont entrés dans cette dite deuxième zone.

2.- Procédé selon la revendication 1 dans lequel au cours de la deuxième étape catalytique de polymérisation au moins 75 % du butène-1 et 55 % du butène-2 ont été convertis en dimères et trimères.

3.- Procédé selon l'une des revendications 1 et 2 dans lequel les deux zones de polymérisation fonctionnent avec une température d'entrée et de sortie comprise entre 45 et 200°C, sous une pression de 0,1 à 5 MPa avec un débit d'hydrocarbures liquides d'environ 0,8 à 2,5 volumes par volume de catalyseur et par heure, les températures d'entrée et de sortie pour la deuxième zone de polymérisation étant chacune supérieure de 30 à 50°C aux températures d'entrée et sortie de la première zone de polymérisation, la pression dans la deuxième zone de polymérisation étant supérieure de 0,8 à 2 MPa à la pression dans la première zone de polymérisation.

4.- Prodécé selon la revendication 1 dans lequel au cours de la première étape, on convertit au moins 85 % de l'isobutène en dimères et trimères de l'isobutène.

5.- Procédé selon l'une des revendications 1 à 4 dans lequel
(voir figure 1) :

a) la charge constituée essentiellement d'une coupe $C_4$ oléfinique renfermant généralement de l'isobutane, du n-butane, des butènes-1 et -2 et de l'isobutène est envoyée par les lignes (1) et (3) dans la dite première zone catalytique de polymérisation (4).

b) l'effluent de la zone (4) est envoyé dans la zone de fractionnement (6) d'où l'on recueille par la conduite (7) une essence constituée de dimères et trimères d'oléfines en $C_4$, et par la conduite (8) un mélange d'isobutène, de butènes-1 et -2 et des hydrocarbures paraffiniques.

c) le mélange de la conduite (8) est partagé en deux parties : la première partie ne représentant pas plus de 30 % en poids du mélange de la conduite (8) étant éliminée ou envoyée au pool essence par une conduite (13), la deuxième partie du dit mélange étant envoyée par la conduite (9) dans la dite deuxième zone catalytique de polymérisation (10),

d) la dite deuxième partie du dit mélange de la conduite 8 est traitée dans la dite zone (10) et

e) l'effluent de la zone catalytique (10) de polymérisation est envoyé par les conduites (11) et (12) dans la zone de fractionnement (6) défini au § (b).

6.- Procédé selon l'une des revendications 1 à 4 dans lequel (voir figure 2) :

a) la charge constituée essentiellement d'une coupe $C_4$ oléfinique renfermant généralement de l'isobutane, du n-butane, des butènes-1 et -2 et de l'isobutène est envoyée par les lignes (1) et (3) dans la dite première zone catalytique de polymérisation (4).

b) l'effluent de la zone (4) est envoyé dans la zone de fractionnement (6) d'où l'on recueille par la conduite (7) une essence constituée de dimères et trimères d'oléfines en $C_4$, et par la conduite (8) un mélange d'isobutène, de butènes-1 et -2 et des hydrocarbures paraffiniques.

4

c) le mélange de la conduite (8) est traité dans une deuxième zone catalytique (10) de polymérisation.

d) l'effluent de la zone (10) est fractionné dans une deuxième zone (12) de fractionnement,

e) on recueille par la ligne 13 des dimères et trimères d' oléfines en C$_4$ et par la ligne (17) un mélange d'hydrocarbures paraffiniques et d'oléfines n'ayant pas réagi.

f) le dit mélange de la ligne (17) est partagé en deux parties, une première partie, ne renfermant pas plus de 30 % en poids du mélange de la conduite (17), étant éliminée ou envoyée au pool essence, par la conduite (14) et une deuxième partie étant recyclée par la ligne (15) dans la deuxième zone catalytique de polymérisation (10).

7.- Procédé selon l'une des revendications 1 à 6 dans lequel on convertit en dimères et trimères au moins 97 % de l'isobutène, au moins 90 % du butène-1 et au moins 80 % du butène-2 de la coupe oléfinique initiale.

PL_unique

**FIG.1**

**FIG.2**

**FIG.3**

### Office européen
### des brevets

## RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | US-A-2 298 330 (ANGELL) <br> * page 2, colonne de gauche, lignes 7-19; page 2, colonne de droite, lignes 32-39, 57-75; page 3, colonne de gauche, lignes 1-27; page 3, colonne de droite, lignes 62-73; revendications 1-5; figures 1,2 * | 1,3-6 | C 10 G 29/00 <br> C 07 C 11/02 |
| A | US-A-3 518 323 (PINE et al.) <br> * colonne 2, lignes 24-57; revendications 1-4,8,11 * | 1,4 | |
| D,A | FR-A-2 515 171 (IFP) <br> * figures; revendication 1 * | 1 | |

**DOMAINES TECHNIQUES RECHERCHES (Int. Cl. ³)**

C 10 G
C 10 L
C 07 C

Le présent rapport de recherche a été établi pour toutes les revendications

| Lieu de la recherche | Date d'achèvement de la recherche | Examinateur |
|---|---|---|
| LA HAYE | 04-10-1984 | DE HERDT O.C.E. |